# EUROPEAN PATENT APPLICATION

(11) **EP 2 465 485 A2**
(43) Date of publication of application: **20.06.2012**
(21) Application number: 10808009.4
(22) Date of filing: 30.07.2010
(51) Int. Cl.: A61K 8/02, A61K 8/34, A61Q 9/02

(54) **LIQUID SHAVING PREPARATION**

(30) Priority: 12.08.2009 ES 200930596
(71) Applicant: Prieto Alonso, José Andrés, 30011 Murcia (ES)
(72) Inventor: Prieto Alonso, José Andrés, 30011 Murcia (ES)
(74) Representative: Illescas, Manuel
(86) International application number: PCT/ES2010/070533
(87) International publication number: WO 2011/018540

(57) **Abstract**

The invention relates to a preparation for topical use, consisting in a solution of cosmetic products that allows their use as a shaving product. The invention comprises the use of deodorised alcohol at a concentration by weight equal to or less than 60%. The use of deodorised alcohol allows the use of perfume aromas at low concentrations or not at all, which does not therefore condition the user in their use of their own personal perfume, allowing them to combine the shaving preparation with the fragrances of their choice, provided by other products. The use of an alcohol concentration of less than 60% by weight involves a reduced feeling of heat on the skin and less irritation of the areas on which the product is applied. Additionally, incorporating a thickener may provide viscosity preparation the preparation in order to facilitate its sticking to the face and a better use thereof.

## Description

### FIELD OF THE INVENTION

This invention relates to a liquid preparation for shaving. More specifically, the invention describes a product consisting in a solution of cosmetic ingredients that combines the effect of shaving soap and aftershave lotion, using deodorised ethyl alcohol in its composition.

### BACKGROUND OF THE INVENTION

The shaving process comprises several stages that usually involve the use of different products, such as shaving foams, gels or soaps, toning aftershave lotions and perfumes, amongst others. These products are usually used separately, using commercial preparations specifically designed for each step during shaving (lathering, shaving, toning and perfume). Despite this being the most extended type of shaving amongst users, we can also find in the state of the art some liquid preparations that combine the effect of the different steps in a single product in order to simplify the shaving process, thus reducing the number of cosmetic solutions required. One of the advantages derived from the use of a liquid shaving product is its capacity to facilitate accurate shaving, since the shaving can be performed over a transparent preparation, which leaves the entire shaving area visible. Another advantage is its lower wear of liquid products on the razor blades, which facilitates the total removal of any shaving waste simply by rinsing the blades in water. The blades thus remain clean and disinfected for longer and do not go rusty. Some examples of this type of liquid shaving products are the preparations described in patents US 4863350, US 2004/247558, US 4065422, US 4279891 and US 4416873. Although these preparations comprise different cosmetic formulations, they may vary in some of their components and/or in the proportions in which these are used, and they all consider the use of a mixture of water and alcohol as the liquid medium for application, with an alcohol concentration of more than 60% by weight of the preparation.

Although, as described above, previous products are capable of satisfying the the need for providing solutions that simultaneously combine the different stages of shaving in a single preparation, the use of alcohol in high concentrations present involves certain drawbacks. On the one hand, the presence of alcohol involves an intense smell that must be covered, normally including perfume aromas in the product composition, in a sufficient amount as to mitigate the smell of the alcohol. This need to use perfumes means that the preparations must all have a characteristic smell, which varies according to the chosen aroma, but which may not necessarily be to the user's liking. Moreover, in many cases the person that is going to use the shaving product does not wish to use any kind of perfume, or to smell of alcohol, and therefore this type of product is not therefore capable of providing the desired function to such users.

As well as the need to use a perfume that counteracts the smell of alcohol, the use of such perfume at a high concentration, above 60% by weight of the preparation, produces in most cases a feeling of heat upon the skin and irritation where it is applied, with the resulting discomfort that this causes the user.

On the other hand, it is also observed in the products of the state of the art mentioned above, that when presented in a liquid state they usually have the drawback of dripping or even trickling from the face during their use, which makes them uncomfortable, cumbersome and dirty to use.

Another drawback of these products is the misting that occurs when applied as a spray. The harmful effect of the alcoholic fumes, specifically the ethyl fumes, is enhanced with the mist of product in suspension that is created.

It is therefore necessary to achieve a liquid shaving product that can combine the effects of soap and aftershave lotion in a single formulation, but without the disadvantages described above of using alcohol at high concentrations and the product being too runny.

This invention prevents said disadvantages by a liquid shaving preparation the formulation of which comprises the use of deodorised alcohol (also known as perfume alcohol) at a concentration by weight equal to or less than 60% of the total weight of the preparation. The use of deodorised alcohol allows the use of perfume aromas at low concentrations or not at all, which does not therefore condition the user in their use of their own personal perfume, allowing them to combine the shaving preparation with the fragrances of their choice, provided by other products. On the other hand, the use of an alcohol concentration equal to or less than 60% by weight involves a reduced feeling of heat on the skin and less irritation where applied, which are more sensitive after shaving, allowing control of the vasodilating effect and consequently providing a more pleasant feeling to the user after shaving. In addition, the reduction of alcohol also entails a proportional reduction in the release of toxic neat alcohol fumes both during the manufacturing process and when used by the user, which entails an additional advantage with respect to the state of the art. On the other hand, in order to avoid an excessively runny preparation that may lead to it dripping, this invention may incorporate a thickening agent, in proportions by weight from 0.1 % to 3%, depending on the type of packaging used. The use of such an agent provides viscosity to the end product, decreasing its flowability and therefore its dripping or runniness, and/or misting during the shaving process.

### OBJECT OF THE INVENTION

One object of this invention is a liquid shaving preparation that combines the effects of shaving soap and aftershave lotion in a single preparation, but without the disadvantages derived from the use of alcohol at a concentration of more than 60% by weight, preventing it from dripping or running, as well as the misting of the preparation during the shaving process.

An additional purpose of the present invention is a liquid shaving preparation the formulation of which comprises the use of deodorised alcohol, preferably rectified ethyl alcohol 96%, also called perfume alcohol. We thus manage to avoid the need for using perfume aromas in the composition of the preparation in order to mitigate the smell of the alcohol itself by using an odourless component: deodorised alcohol.

One object of this invention is a liquid shaving preparation the formulation of which comprises the use of deodorised alcohol at a concentration by weight equal to or less than 60% of the total weight of the preparation. We thus manage to control the vasodilating effect of alcohol on the skin when used at high concentrations, preventing the feeling of heat or irritation, and also minimising the emission of neat alcohol fumes and their harmful effect when inhaled.

Another object of this invention is a liquid shaving preparation that consists in, amongst other components, a mixture of alcohol and water where the water used may optionally be distilled water.

Also object of this invention is a liquid shaving preparation containing cosmetic industry agents for skin care, including, for example, glycerin (emollient and moisturising effect), pH regulators (NaOH and citric acid, for example), triethanolamine (conditioner, lubricant and pH regulator), menthol (toning effect when added in low concentrations, such that its fragrance does not last on the skin after application, but maintaining the feeling of freshness; at a concentration of not more than 1 % by weight of the total weight of the preparation, for example), perfume aromas (said aromas understood as natural or synthetic essential oils, which provide aroma), one or more active ingredients with a relaxing, sedative and cell activity activating effect on the skin, such as, for example, liquids comprising milk proteins (International Nomenclature of Cosmetic Ingredients: "Lactis Proteinum") or its commercial presentations, (such as, for example, "Lactokine Fluid"), and/or colouring substances. In addition, the liquid preparation may also contain one or more nutritional substances, such as collagen, for example.

Another object of this invention is a process for manufacturing a liquid shaving preparation.

The liquid shaving preparation object of the invention can be presented in containers with different kinds of nozzle, without requiring the use of the metal containers used for packaging other products of the state of the art, such as shaving foam, which require pressurised packaging. Metal containers are difficult to recycle, whereas the liquid shaving preparation may be packaged in containers manufactured in plastic or bioplastic materials, for example.

An object of the invention is therefore a liquid shaving preparation that allows preventing the use of pressurised gases such as methylbutane and/or methylpropane in its manufacture, since these may involve health risks due to their flammable properties, or due to the potential damage they may cause when inhaled (lung damage, drowsiness, vertigo) and when applied repeatedly on the skin (dry or cracked skin).

Another object of the invention is a shaving preparation that can be presented in wet towels and preserved in sealed bags.

Another object of this invention is a shaving preparation incorporating in its composition a thickening agent that is transparent when extended over the skin. Optionally, said preparation may be opaline inside the packaging.

Another object of this invention is the use of deodorised alcohol in shaving products, where such products may optionally be presented in liquid preparations.

Other characteristics and advantages of this invention shall be derived from the description of the invention provided below, and from the illustrative embodiment and the accompanying figures.

### DESCRIPTION OF THE DRAWINGS

Figure 1 schematically represents the manufacturing process associated to a preferred embodiment of the present invention.
Figure 2 schematically represents the manufacturing process associated to a preferred embodiment of the present invention.

### DETAILED DESCRIPTION OF THE INVENTION

This invention consists of an alcohol and water based liquid preparation with a preferably transparent or translucent appearance, indicated for use as a shaving product and that serves as a vehicle for the incorporation of different ingredients that provide, amongst others, the following effects: emollient, lubricant, moisturising, disinfectant, sedative, repair and nutrient. It is also possible to add colorant and/or perfume essence substances to the preparation in sufficient amounts according to the aesthetic and commercial criteria to be applied.

The preparation of this invention essentially consists in USP deodorised ethyl alcohol as an active ingredient, dissolved in water, to be used as the vehicle into which to incorporate different cosmetic agents to the formulation in order to provide skin care, as well as other ingredients and excipients adapted to each kind of preparation. In a preferred embodiment of the invention, the shaving product optionally incorporates one or more agents with emollient and moisturising properties (such as glycerin, for example), one or more agents with toning properties (including menthol, for example, added in low concentrations so that its fragrance does not last on the skin after application), one or more agents with softener, lubricant and pH-regulating properties (including triethanolamine, for example) and one or more agents with nutrient properties (such as collagen, for example), intended to provide a greater feeling of freshness and softness during the shaving process. In addition, the shaving preparation comprises one or more thickeners that provide viscosity (such as hydroxyethylcellulose) and optionally one or more active ingredients with a sedative, relaxing and invigorating effect on the skin (such as milk proteins).

In embodiments of the invention comprising the use of perfume aromas, it is also possible to use one or more fixing agents in the preparation formulation in order to prevent the volatilisation of the essential oils and a longer lasting perfume. Fixing agents are substances that are less volatile than the perfumes oils used, which reduce the evaporation rate of the aromatic components. The types of fixing agents commonly used in the state of the art comprise animal secretions, resinous products and/or synthetic chemical products. One commonly used fixing agent is polypropylene glycol (PPG), which in one possible embodiment of the present invention is mixed in a proportion of 1 to 16 grams per litre of deodorised alcohol used. The use of a fixing agent allows reducing the proportion of aromas used, which is advantageous since said aromas are usually quite expensive components.

For one preferred embodiment of the invention described above, the liquid shaving preparation has the following composition, for 1000 grams of preparation:

| **Ingredient** | **% by weight** |
|---|---|
| Deodorised ethyl alcohol (USP) | ≤ 60 |
| Glycerin | 3 |
| Menthol | 0,1 |
| Aroma | To taste |
| Colour | To taste |
| Triethanolamine | ≤ 0,4 |
| Distilled water | Sufficient amount to complete the mixture |

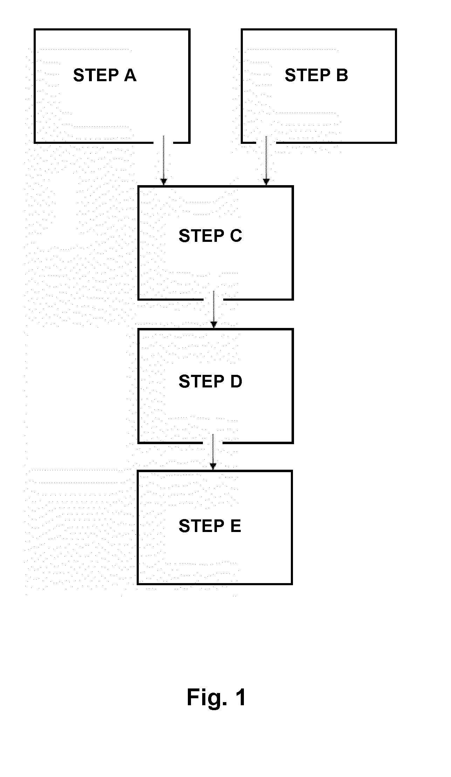

In this preferred embodiment of the invention, the manufacture of the liquid shaving preparation is performed following the procedure represented diagrammatically in Figure 1 and described below, in the proportions required to obtain 1000 grams of the preparation:
- Step A - Mix:
   - Deodorised ethyl alcohol (USP) - Up to 600 ml.
   - Glycerin - 30 gr.
   - Aroma (to taste) - 20 gr.
   - Menthol - 1 gr.
- Step B: - Mix:
   - Distilled water - 250 ml.
   - Topical colouring (to taste), ensuring that the mixture remains transparent - Up to 1 gr.
- Step C - Mix:
   - Step A - 651 gr.
   - Step B: - 50 gr.
   - Distilled water - 295 ml.
   - Triethanolamine (with a pH value between 6.5 and 7) - 4 gr.
- Step D: - Allow the mixture of Step C to steep for approximately 20 days at a stable temperature of close to 4°C.
- Step E: - Filter and bottle the preparation obtained from Step D. Magnesium carbonate may be added to favour filtration, due to its waste precipitating properties.

In other embodiments of the above procedure, it is also possible to skip the steeping step (Step D). Also, in cases in which the procedure comprises the use of perfume aromas (Step A), it is also possible to optionally incorporate into the preparation composition between 1 and 16 grams of a fixing agent (preferably polypropylene glycol) per litre of deodorised alcohol used.

For another preferred embodiment of the invention, the liquid shaving preparation has the following composition, for 1000 grams of preparation:

| **Ingredient** | **% by weight** |
|---|---|
| Deodorised ethyl alcohol (USP) | ≤ 60 |
| Glycerin | 3 |
| Menthol | 0,1 |
| Aroma | To taste |
| Colour | To taste |
| Milk Protein (Lactokine Fluid) | 2 |
| Hydroxyethyl cellulose | 1,3 |
| 10% Caustic Soda (NaOH) | Sufficient amount |
| 10% citric acid monohydrate | Sufficient amount |
| Distilled water | Sufficient amount to complete the mixture |

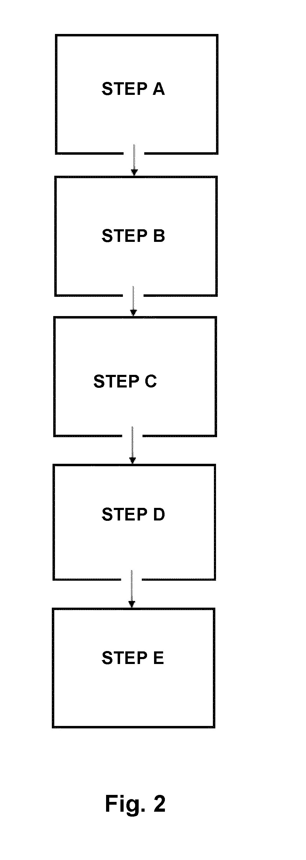

In this preferred embodiment of the invention, the manufacture of the liquid shaving preparation is performed following the procedure represented diagrammatically in Figure 2 and described below to obtain 1000 grams of the preparation:
- Step A - Mix:
   - Distilled water - 250 ml.
   - Cosmetic colouring (to taste) - Up to 1 gr.
- Step B - Mix and dissolve in a container with stirring:
   - Step A - 50 gr.
   - Deodorised ethyl alcohol (USP) - Up to 600 ml.
   - Glycerin - 30 gr.
   - Aroma (to taste) - 20 gr.
   - Menthol - 1 gr.
   - Milk Protein (Lactokine Fluid) - 20 gr.
   - Distilled water. From 264 ml.
- Step C - Mix and disperse in a container with turbo stirring:
   - Step B: - 986 gr.
   - 10% Caustic soda (NaOH), sufficient amount to reach pH-9.
   - Hydroxyethyl cellulose (to taste). Up to 13 gr.

Allow the mixture obtained to stand for a few minutes until it releases any air it may have trapped, and stir again until observing complete polymerisation of the cellulose.
- Step D - Neutralise the mixture obtained in Step C with 10% citric acid monohydrate in a sufficient amount to reach pH 5-6.
- Step E - Package.

In cases in which the procedure comprises the use of perfume aromas (Step B), it is also possible to optionally incorporate into the preparation composition between 1 and 16 grams of a fixing agent (preferably polypropylene glycol) per litre of deodorised alcohol used.

In different preferred embodiments of the invention, the shaving preparation may be presented in bags and in different types of containers, applied by blasting or as a spray, without requiring the use of metal containers that are difficult to recycle, since they can be packaged in containers manufactured with plastic or bioplastic materials. This possibility for packaging avoids the use of pressurised gases such as methylbutane and/or methylpropane in its manufacture, which may involve health risks.

In a preferred embodiment of the invention, the shaving preparation may be presented as wet towels soaked in the product and kept in sealed bags.

The use of deodorised alcohol as described in this invention may also be generalised to other shaving products not formulated in a liquid medium, such as soaps, foams or gels, providing such products with the properties described above of reducing the smell of alcohol and the irritation caused on the skin.

The embodiments described for this invention must not be considered limiting against variations in its composition or in the proportions used to manufacture the product, provided that said variations do not alter the essence or the object of the invention.

## Claims

1. A liquid shaving preparation **characterised in that** it comprises deodorised alcohol as one of its components.

2. A liquid shaving preparation according to claim 1, **characterised in that** the deodorised alcohol content in its composition is equal to or less than 60% by weight of said preparation.

3. A liquid shaving preparation according to any of claims 1-2, **characterised in that** it additionally comprises water.

4. A liquid shaving preparation according to claim 3, **characterised in that** its proportion of water to alcohol is 3.5:6.

5. A liquid shaving preparation according to any of claims 3-4, **characterised in that** it the water it comprises is distilled water.

6. A liquid shaving preparation according to any of claims 1-5, **characterised in that** it additionally comprises at least one emollient and/or moisturising substance.

7. A liquid shaving preparation according to claim 6, **characterised in that** the emollient and/or moisturising substance is glycerin.

8. A liquid shaving preparation according to any of claims 1-7, **characterised in that** it additionally comprises at least one toning substance.

9. A liquid shaving preparation according to claim 8, **characterised in that** the toning substance is menthol.

10. A liquid shaving preparation according to any of claims 1-9, **characterised in that** it additionally comprises at least one pH regulating substance.

11. A liquid shaving preparation according to claim 10, **characterised in that** the pH regulating substance is one or more of the following: triethanolamine, caustic soda and/or citric acid.

12. A liquid shaving preparation according to any of claims 1-11, **characterised in that** it additionally comprises at least one aroma.

13. A liquid shaving preparation according to claim 12, **characterised in that** the aroma is a natural or synthetic perfume essential oil.

14. A liquid shaving preparation according to any of claims 1-13, **characterised in that** it additionally comprises at least one substance with a sedative, relaxing and invigorating effect on the skin.

15. A viscous liquid shaving preparation according to claim 14, **characterised in that** the substance with the sedative, relaxing and invigorating effect on the skin is a milk protein.

16. A viscous liquid shaving preparation according to any of claims 1-15, **characterised in that** it additionally comprises at least one thickener that provides viscosity to the preparation.

17. A viscous liquid shaving preparation according to claim 16, **characterised in that** the thickener is hydroxyethyl cellulose.

18. A liquid shaving preparation according to any of claims 1-17, **characterised in that** it additionally comprises at least one nutrient.

19. A liquid shaving preparation according to claim 18, **characterised in that** the nutrient is collagen.

20. A liquid shaving preparation according to any of claims 1-19, **characterised in that** it additionally comprises at least one colouring substance.

21. A liquid shaving preparation according to any of claims 1-20, **characterised in that** its composition, for 1000 gr of the preparation, comprises the following substances:
a) 600 grams of deodorised ethyl alcohol (USP).
b) 30 grams of glycerin.
c) 1 gram of menthol.
d) 4 grams of triethanolamine.
e) Perfume aroma to taste.
f) Colouring to taste.
g) Distilled water in a sufficient amount to complete 1000 grams of the preparation.

22. A liquid shaving preparation according to any of claims 1-20, **characterised in that** its composition, for 1000 gr of the preparation, comprises the following substances:
a) 600 grams of deodorised ethyl alcohol (USP).
b) 30 grams of glycerin.
c) 1 gram of menthol.
d) 20 grams of a cosmetic active ingredient based on milk proteins.
e) Perfume aroma to taste.
f) Colouring to taste.
g) A sufficient amount of soda (NaOH) at a concentration of 10% by weight.
h) Up to 30 grams of hydroxyethyl cellulose.
i) A sufficient amount of 10% citric acid monohydrate.
j) Distilled water in a sufficient amount to complete 1000 grams of the preparation.

23. A liquid shaving preparation according to any of claims 1-22, **characterised in that** it additionally comprises at least one fixing agent.

24. A liquid shaving preparation according to claim 23, **characterised in that** the fixing agent Is polypropylene glycol.

25. A liquid shaving preparation according to any of claims 1-24, **characterised in that** it is presented packaged in a plastic or bioplastic container comprising means for distributing or pouring said preparation, either via blasting or spraying or in bag.

26. A liquid shaving preparation according to any of claims 1-24, **characterised in that** it is presented in wet towels soaked in the preparation, said wet towels being kept in a container such as a bag or a box, for example.

27. A procedure for preparing a liquid shaving preparation according to any of claims 1-26, **characterised in that** it comprises the following steps:
- Step A: A first solution is prepared consisting in the mixture of deodorised ethyl alcohol, and optionally at least one emollient and/or moisturising substance, at least one toning substance and at least one aroma.
- Step B: A second solution is prepared consisting in a mixture of water, and optionally a colouring substance.
- Step C: A third solution is prepared that consists in the mixture of the solution obtained in Step A and an amount to taste of the solution obtained in Step B, sufficient to provide the intended colour, together with optionally at least one softener and/or lubricant and more water, maintaining the mixture at a pH between 6.5 and 7.
- Step D: The solution obtained in Step C is allowed to steep for approximately 20 days.
- Step E: The preparation obtained in Step D is filtered until producing a product that is free from impurities, and is then packaged.

28. A procedure for preparing a liquid shaving preparation according to any of claims 1-26, **characterised in that** it comprises the following steps:
- Step A: A first solution is prepared consisting in the mixture of deodorised ethyl alcohol, and optionally at least one emollient and/or moisturising substance, at least one toning substance and at least one aroma.
- Step B: A second solution is prepared consisting in a mixture of water, and optionally a colouring substance.
- Step C: A third solution is prepared that consists in the mixture of the solution obtained in Step A and an amount to taste of the solution obtained in Step B, sufficient to provide the intended colour, together with optionally at least one softener and/or lubricant and more water, maintaining the mixture at a pH between 6.5 and 7.
- Step D: The preparation obtained in Step C is filtered until producing a product that is free from impurities, and is then packaged.

29. A procedure according to any of claims 27-28, where Step A additionally comprises adding between 1 and 16 grams of polypropylene glycol per litre of deodorised alcohol used.

30. A procedure for preparing a liquid shaving preparation according to any of claims 1-26, **characterised in that** it comprises the following steps:
- Step A: A first solution is prepared consisting in a mixture of water, and optionally a cosmetic colouring substance.
- Step B: A second solution is mixed consisting in the mixture of a sufficient amount of the solution obtained in step A to achieve the intended colour, and deodorised ethyl alcohol, optionally together with at least one emollient and/or moisturising substance, at least one toning substance, at least one aroma, at least one active cosmetic substance with a sedative, relaxing and cell activity invigorating effect on the skin, and more distilled water.
- Step C: Perfectly disperse at least one cosmetic thickener with stirring in the mixture obtained in Step B according to the degree of viscosity required. Previously adjust the pH if necessary. Allow the mixture to stand long enough until it releases any air that may have been trapped during stirring. Stir the mixture again until observing complete dispersion of the thickener.
- Step D: Adjust the pH of the mixture obtained from Step C until obtaining a pH between 5 and 6.
- Step E: Package the mixture obtained in Step D.

31. A procedure according to claim 30, where Step B additionally comprises adding between 1 and 16 grams of polypropylene glycol per litre of deodorised alcohol used.

32. Use of a preparation according to any of claims 1-26 in the preparation of shaving products.
